# EUROPEAN PATENT APPLICATION

(11) **EP 3 674 413 A1**
(43) Date of publication of application: **01.07.2020**
(21) Application number: 18902605.7
(22) Date of filing: 18.07.2018
(51) Int. Cl.: C12Q 1/68

(54) **PROBE AND METHOD FOR HIGH-THROUGHPUT SEQUENCING TARGETED CAPTURE TARGET REGION USED FOR DETECTING GENE MUTATIONS AS WELL AS KNOWN AND UNKNOWN GENE FUSION TYPES**

(30) Priority: 26.01.2018 CN 201810076496
(71) Applicant: Amoy Diagnostics Co., Ltd, Xiamen, Fujian 361027 (CN)
(72) Inventor: ZHANG, Linhua, Xiamen, Fujian 361027 (CN); LI, Xuchao, Xiamen, Fujian 361027 (CN); JIN, Baolei, Xiamen, Fujian 361027 (CN); SHI, Weijie, Xiamen, Fujian 361027 (CN); GE, Huijuan, Xiamen, Fujian 361027 (CN); RUAN, Li, Xiamen, Fujian 361027 (CN); ZHENG, Limou, Xiamen, Fujian 361027 (CN)
(74) Representative: Casalonga
(86) International application number: PCT/CN2018/096080
(87) International publication number: WO 2019/144582

(57) **Abstract**

The present invention discloses a probe and method for high-throughput sequencing target-captured target regions for detecting gene mutations and known and unknown gene fusion types. The probe includes an extension probe and a connection probe, the extension probe including an extension arm, a sequencing tag sequence 1 region, a protection structure 1 region, wherein a unique identification (UID) sequence 1 region may be further provided between the extension arm and the sequencing tag sequence 1 region, and the connection probe including a ligation arm, a sequencing tag sequence 2 region and a protection structure 2 region, wherein a UID sequence 2 region may be further provided between the ligation arm and the sequencing tag sequence 2 region. The present invention has advantages of simple operation, short experimental period, low costs, high sensitivity, high specificity, and capability of directly detecting known and unknown gene fusion types at a level of DNA.

## Description

### Technical field

The present invention relates to a biotechnology sequencing field, and in particular, to a probe and method for high-throughput sequencing target-captured target regions used for detecting gene mutations and known and unknown gene fusion types.

### Background art

Target region targeted sequencing is a research strategy that performs high-throughput sequencing after capturing and enriching a target region of interest. With the completion of more and more genome sequencing projects, customized target region sequencing has gradually become the preferred method for the researchers with respect to specific research regions. In comparison with the whole genome sequencing, the target region targeted sequencing has main advantages lying in sequencing a specific region, significantly improving researching efficiency on a specific target region, greatly reducing research costs, producing more accurate results, and easily discovering low-frequency mutations. The technology may be applied to identifying and studying complex diseases, and has great application potential in clinical diagnosis and drug development. The current common methods for target capturing a target region mainly include the following two types: multiple polymerase chain reaction (PCR) and hybrid capturing.

The multiple PCR has advantages of high sensitivity, good specificity, good reproducibility, easy operation and low price etc. However, a large amount of primers in a same reaction tube easily cause problems such as generating a large amount of primer dimers, the primer cross-amplification, and partial amplicons failure to be amplified. The multiple PCR cannot detect the unknown gene fusion types, and the method is only applicable to capturing the relatively smaller target region.

The hybrid capturing can capture a whole exome and even the bigger target region, and has advantages of good scalability and being able to detect low-frequency mutations and unknown gene fusion types. However, the hybrid capturing has disadvantages that the operation process is extremely complicated, the cycle is long, the cost is high, and more special instruments and equipment are required. These disadvantages have limited its application in practical clinical diagnosis to a certain extent.

### Summary

The present invention aims to provide a method for high-throughput sequencing target-captured target region and having the advantages of simple operation, short experimental period, low costs, high sensitivity, high specificity, and being capable of directly detecting known and unknown gene fusion types at a level of DNA.

In order to implement the above purpose, the present invention provides a probe for detecting gene mutations as well as known and unknown gene fusion types, which is characterized in including an extension probe and a connection probe, wherein extension probe consists of following structures:
an extension arm: a sequence complementarily bonded with a nucleic acid template and having a length of 16-50 nt;
a sequencing tag sequence 1 region: used for a Tag1 sequence sequenced on a sequencing platform, and being a sequence or its complementary sequence on transposon Adapters or linkers in any library building kit of the sequencing platform;
a protection structure 1 region: being a 3 to 17 phosphorothioate modifications sequence or a spacer arm, wherein the 3 to 17 phosphorothioate modifications sequence refers to phosphorothioate modifying 3 to 17 bases at a 5' end of the sequencing tag sequence 1 region or phosphorothioate modifying 3 to 17 bases of any section, used for protecting the probe and preventing exonuclease digestion, and preferably being a 3 to 7 phosphorothioate modifications sequence or a spacer arm; and
a UID sequence 1 region further provided between the extension arm of the extension probe and the sequencing tag sequence 1 region, preferably, the UID sequence 1 region consisting of 3 to 12 random bases for distinguishing tag sequences of an original template; and
the connection probe consists of following structures:
   a ligation arm: a sequence complementarily bonded with a nucleic acid template and having a length of 16-50 nt, wherein phosphorylation modification is performed on a 5' end of the ligation arm, or phosphorylation modification is voluntarily performed on the 5' end through phosphorylation reaction when the probe is synthesized;
   a sequencing tag sequence 2 region: used for a Tag2 sequence sequenced on a sequencing platform, and being a sequence or its complementary sequence on transposon Adapters or linkers in any library building kit of the sequencing platform;
   a protection structure 2 region: being a 3 to 19 phosphorothioate modifications sequence or a spacer arm, used for protecting the probe and preventing exonuclease digestion, wherein the 3 to 19 phosphorothioate modifications sequence refers to phosphorothioate modifying 3 to 19 bases at a 3' end of the sequencing tag sequence 2 region or phosphorothioate modifying 3 to 19 bases of any section, and preferably being a 3 to 7 phosphorothioate modifications sequence or a spacer arm.

On another aspect, the present invention provides another probe for detecting gene mutations as well as known and unknown gene fusion types, which is characterized in including an extension probe and a connection probe, wherein the extension probe consists of following structures:
an extension arm: a sequence complementarily bonded with a nucleic acid template and having a length of 16-50 nt;
a sequencing tag sequence 1 region: used for a Tag1 sequence sequenced on a sequencing platform, and being a sequence or its complementary sequence on transposon Adapters or linkers in any library building kit of the sequencing platform;
a protection structure 1 region: being a 3 to 17 phosphorothioate modifications sequence or a spacer arm, wherein the 3 to 17 phosphorothioate modifications sequence refers to phosphorothioate modifying 3 to 17 bases at a 5' end of the sequencing tag sequence 1 region or phosphorothioate modifying 3 to 17 bases of any section, used for protecting the probe and preventing exonuclease digestion, and preferably being a 3 to 7 phosphorothioate modifications sequence or a spacer arm; and
the connection probe consists of following structures:
   a ligation arm: a sequence complementarily bonded with a nucleic acid template and having a length of 16-50 nt, wherein phosphorylation modification is performed on a 5' end of the ligation arm, or phosphorylation modification is voluntarily performed on the 5' end through phosphorylation reaction when the probe is synthesized;
   a sequencing tag sequence 2 region: used for a Tag2 sequence sequenced on a sequencing platform, and being a sequence or its complementary sequence on transposon Adapters or linkers in any library building kit of the sequencing platform;
   a UID sequence 2 region further provided between the ligation arm of the connection probe and the sequencing tag sequence 2 region, preferably, the UID sequence 2 region consisting of 3 to 12 random bases for distinguishing tag sequences of an original template; and
   a protection structure 2 region: being a 3 to 19 phosphorothioate modifications sequence or a spacer arm, used for protecting the probe and preventing exonuclease digestion, wherein the 3 to 19 phosphorothioate modifications sequence refers to phosphorothioate modifying 3 to 19 bases at a 3' end of the sequencing tag sequence 2 region or phosphorothioate modifying 3 to 19 bases of any section, and preferably being a 3 to 7 phosphorothioate modifications sequence or a spacer arm.

On another aspect, the present invention provides another probe for detecting gene mutations as well as known and unknown gene fusion types, which is characterized in including an extension probe and a connection probe, wherein the extension probe consists of following structures:
an extension arm: a sequence complementarily bonded with a nucleic acid template and having a length of 16-50 nt;
a sequencing tag sequence 1 region: used for a Tag1 sequence sequenced on a sequencing platform, and being a sequence or its complementary sequence on transposon Adapters or linkers in any library building kit of the sequencing platform;
a UID sequence 1 region further provided between the extension arm of the extension probe and the sequencing tag sequence 1 region, preferably, the UID sequence 1 region consisting of 3 to 12 random bases for distinguishing tag sequences of an original template; and
a protection structure 1 region: being a 3 to 17 phosphorothioate modifications sequence or a spacer arm, wherein the 3 to 17 phosphorothioate modifications sequence refers to phosphorothioate modifying 3 to 17 bases at a 5' end of the sequencing tag sequence 1 region or phosphorothioate modifying 3 to 17 bases of any section, used for protecting the probe and preventing exonuclease digestion, and preferably being a 3 to 7 phosphorothioate modifications sequence or a spacer arm; and
the connection probe consists of following structures:
   a ligation arm: a sequence complementarily bonded with a nucleic acid template and having a length of 16-50 nt, wherein phosphorylation modification is performed on a 5' end of the ligation arm, or phosphorylation modification is voluntarily performed on the 5' end through phosphorylation reaction when the probe is synthesized;
   a sequencing tag sequence 2 region: used for a Tag2 sequence sequenced on a sequencing platform, and being a sequence or its complementary sequence on transposon Adapters or linkers in any library building kit of the sequencing platform;
   a UID sequence 2 region further provided between the ligation arm of the connection probe and the sequencing tag sequence 2 region, preferably, the UID sequence 2 region consisting of 3 to 12 random bases for distinguishing tag sequences of an original template; and
   a protection structure 2 region: being a 3 to 19 phosphorothioate modifications sequence or a spacer arm, used for protecting the probe and preventing exonuclease digestion, wherein the 3 to 19 phosphorothioate modifications sequence refers to phosphorothioate modifying 3 to 19 bases at a 3' end of the sequencing tag sequence 2 region or phosphorothioate modifying 3 to 19 bases of any section, and preferably being a 3 to 7 phosphorothioate modifications sequence or a spacer arm.

Furthermore, the sequencing tag sequence 1 is a sequence from which 0 to 18 bases is reduced at the 5' end of SEQ ID NO:1.

Furthermore, the sequencing tag sequence 2 is a sequence from which 0 to 16 bases are reduced at the 3' end of a reverse complement sequence of SEQ ID NO:2, wherein the SEQ ID NO:2 is a section of a sequence of Nextera Transposase Adapters Read 2 in Illumina Nextera Library Prep Kits.

The present invention further provides an application of any probe for detecting gene mutations as well as known and unknown gene fusion types to high-throughput sequencing a target-captured target region used for detecting known and unknown gene fusion types.

The present invention further provides a method applicable to high-throughput sequencing a target-captured target region used for detecting gene mutations as well as known and unknown gene fusion types,
preferably, steps being:
hybridizing a probe with a nucleic acid sample;
extending and ligase ligating reaction: after both the extension probe and the connection probe are anchored to a target region of the nucleic acid sample, extending from the 3' end of the extension probe to the 5' end of the connection probe using a polymerizing function of a DNA polymerase, and a ligase ligating to obtain a ligation product;
or simultaneously performing the hybridizing of the probe with the nucleic acid sample, the extending and the ligation reaction, when the 3' end of the extension probe is phosphorothioate modified and a Tm value of the ligation arm is higher than a Tm value of the extension arm;
exonuclease digesting the linear probe and nucleic acid: digesting the probe and the nucleic acid sample by using a single-stranded exonuclease and a double-stranded exonuclease and only leaving the legation product in the previous step, in order to eliminate the effects of the nucleic acid sample and the remaining probe on subsequent PCR amplification;

PCR amplifying, wherein an upstream primer structure sequentially comprises a sequence region for cluster generation, and an index sequence region for sequencing starting from the 5' end; and a full-length sequence region reversely complementary paired with a sequencing Tag sequence 1, wherein preferably, the 5' end, or the 3' end, or the 5' and 3' ends of the upstream primer are phosphorothioate modified; and wherein a downstream primer structure sequentially comprises a sequence region for cluster generation, and an index sequence region for sequencing starting from the 5' end; and a sequencing tag sequence 2 full-length sequence region, wherein preferably, the 5' end, or the 3' end, or the 5' and 3' ends of the downstream primer are phosphorothioate modified;
library purifying: purifying a library by means of magnetic beads, gel extraction after electrophoresis or column purification; and
high-throughput sequencing.

Furthermore, in the hybridizing the probe with the nucleic acid sample, the nucleic acid sample is DNA, RNA, cDNA obtained by RNA reverse transcription, a mixture of DNA and RNA, or a mixture of DNR and cDNA.

Furthermore, the PCR amplified upstream primer is SEQ ID NO:3, or what is shown by a sequence after the 5' end thereof is phosphorothioate modified, or the 3' end thereof is phosphorothioate modified, or the 5' and 3' ends thereof are phosphorothioate modified.

Furthermore, the PCR amplified downstream primer is SEQ ID NO:4, or what is shown by a sequence after the 5' end thereof is phosphorothioate modified, or the 3' end thereof is phosphorothioate modified, or the 5' and 3' ends thereof are phosphorothioate modified.
SEQ ID NO:1 is 5'-ACACTCTTTCCCTACACGACGCTCTTCCGATCT-3';
SEQ ID NO:2 is 5'-CTGTCTCTTATACACATCTCCGAGCCCACGAGAC-3'.

The method of the present invention includes the following parts:

### 1. Hybridizing Probe With Nucleic Acid Sample:

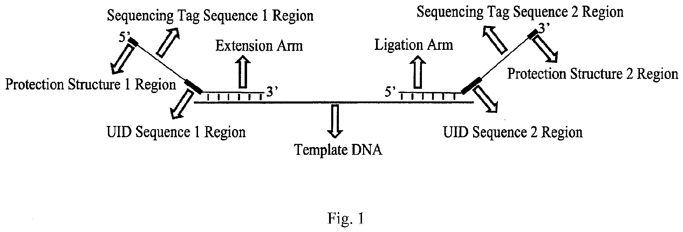

Hybridizing a probe with a nucleic acid sample refers to that the probe has a sequence complementarily paired with a target region of a single-stranded nucleic acid sample and they are hybridized under an appropriate hybridization system and temperature. The nucleic acid sample may be DNA, RNA or cDNA obtained by RNA reverse transcription, or may be a mixture of DNA and cDNA or a mixture of DNR and RNA. FIG. 1 illustrates a probe structure. The probe may be made from a variety of synthetic methods, or may be modified by molecular biological means after the synthesis. The probe includes two types: a connection probe and an extension probe.

### 2. Extending and Ligase Ligating Reaction:

After both the extension probe and the connection probe are anchored to a target region, the extending is performed from the 3' end of the extension probe to the 5' end of the connection probe using a polymerizing function of a DNA polymerase. A High-fidelity DNA polymerase is preferably selected. Then a cut is ligated using a ligase. Certainly, the hybridizing, the extending and the ligation reaction may also be performed simultaneously.

### 3. Exonuclease Digesting Linear Probe and Nucleic Acid:

The probe and the nucleic acid sample are digested by using a single-stranded exonuclease and a double-stranded exonuclease and only the legation product is left in the previous step, in order to eliminate the effects of the nucleic acid sample and the remaining probe on subsequent PCR amplification.

### 4. PCR Amplifying:

The library is amplified by using upstream and downstream primers (5'CAAGCAGAAGACGGCATACGAGATTTCTGCCTGTCTCGTGGGCTCGGAGATGTGTAT AAGAGACAG3', SEQ ID NO:3 and 5'AATGATACGGCGACCACCGAGATCTACACATAGAGGCACACTCTTTCCCTACACGACG CTCTTCCGATCT3', SEQ ID NO:4) containing structures identical with or complementary to the sequencing tag sequences in the probe structure.

The underlined portion is an index sequence for distinguishing tags of different libraries when sequencing mixed libraries, and the sequence is changeable. Please note that a 5' end and a 3' end of the PCR primer may be phosphorothioate modified, and an exonuclease and a PCR reagent may be mixed.

### 5. Library Purifying:

In order to remove impurities such as enzymes, deoxyribonucleoside triphosphates (dNTPs), ions, primers, primer dimers and the like from the library, the library is purified by using Agencourt AMPure XP magnetic beads of Beckman company, and may also be purified by using other means such as gel extraction after electrophoresis or column purification, etc.

### 6. High-Throughput Sequencing:

The prepared library is sequenced on a high-throughput sequencer after the concentration and fragment quality control are qualified. The library sequencing method of the present invention includes, but is not limited to, a reversible end termination sequencing method, and other sequencing platforms may also be adopted. The sequencing type may be single-ended or double-ended. In embodiments of the present invention, the sequencing platform is an Illumina sequencing platform, and the sequencing type is double-end.

In comparison with the current conventional target capturing methods, the advantages of the present invention mainly lie in:
1. the present invention has characteristics of simple operation, short experimental period, low costs, high sensitivity, high specificity and the like; and
2. known and unknown gene fusion types may be directly detected at a level of DNA.

### Brief Description Of The Drawings

FIG. 1 is a diagram illustrating a probe structure and hybridization of the probe with a template.
FIG. 2 is a diagram illustrating a probe structure and hybridization of the probe with a template.
FIG. 3 is a diagram illustrating a probe structure and hybridization of the probe with a template.

### Detailed Description of the Embodiments

The embodiments of the present invention are described in details. Examples of the embodiments are shown in the drawings, wherein the same or similar reference signs indicate the same or similar components or components having the same or similar functions from beginning to end. The embodiments described by referring to the drawings below are exemplary, and aim at explaining the present invention, but cannot be understood as limitations to the present invention. The embodiments in which specific techniques or conditions are not noted, are performed according to the techniques or conditions described in the documents within the prior art or according to the product specifications. The used reagents or instruments that are not marked with manufacturers are all conventional products that can be obtained through market shopping.

The probes and primers in the following embodiments are all synthesized by Sangon Biotech (Shanghai) Co., Ltd. H1650 cell line may be purchased from companies such as Shanghai Zeye Biological Technology Co. Ltd., etc. H2228 and H3122 cell lines may be purchased from companies such as Shanghai Zeye Biological Technology Co. Ltd., Shanghai Jihe Biological Technology Co. Ltd. etc.

In the following embodiments, N may denote any base of A, T, G or C.

### Embodiment 1: Targeting to capture human EGFR gene

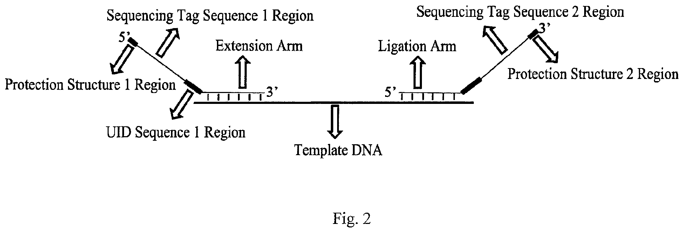

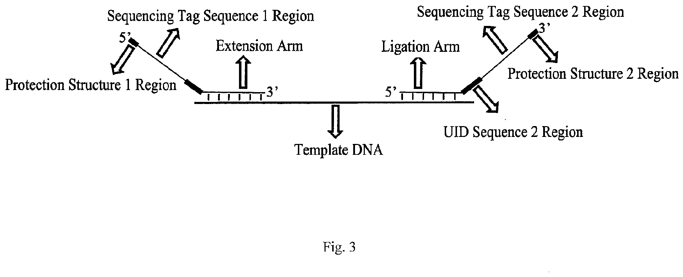

In the embodiment, two pairs of probes of exons 18 and 19 of the target human *EGFR* gene are used, the probes are mixed in equal moles, and the probe structures are shown in FIG. 2 and FIG. 3.

A probe sequence is provided as follows:
P1-a (extension probe) as shown by SEQ ID NO:5:
   5'A-s-C-s-G-s-CTCTTCCGATCTACCCTTGTCTCTGTGT
P1-b (connection probe) as shown by SEQ ID NO:6:
   5'P-AGCCCAGAGTCCTTGCNNNNNNNNNNNNCTGTCTCTTATACAC-s-A-s-T-s-C
P2-a (extension probe) as shown by SEQ ID NO:7:
   5'A-s-C-s-G-s-CTCTTCCGATCTNNNNNNNNNNNNCAGCATGTGGCACCAT
P2-b (connection probe) as shown by SEQ ID NO:8:
   5'P-ACCTTTTCTCATGTCTCTGTCTCTTATACAC-s-A-s-T-s-C.

s denotes phosphorothioate modification, and P denotes phosphorylation modification; the underlined portion in P1-a is an Illumina sequencing tag sequence 1, and a portion underlined with a dotted line is an extension arm; twelve Ns in P1-b denotes a UID sequence 2 region, a portion underlined with a double line is a ligation arm; twelve Ns in P2-a denotes a UID sequence 1 region, a portion underlined with a dotted line is an extension arm; a portion underlined with a double line is a ligation arm; a portion underlined with a wavy line is an Illumina sequencing tag sequence 2; and 3 phosphorothioate modifications in P1-a are a protection structure 1 region, and 3 phosphorothioate modifications in P1-b are a protection structure 2 region.
Steps: 1. Hybridizing Probe With Nucleic Acid Sample: Hybridization reaction system is as shown in Table 1.

**Table 1 Hybridization Reaction System of Embodiment 1**

| Reagent | Volume |
|---|---|
| Purified Water | 6µL |
| 50 ng/µL H1650 cell line DNA | 2µL |
| 3 × 10⁻³µM probe mixture (i.e., a mixture of SEQ ID NO:5-8) | 1µL |
| 10 × Ampligase Buffer (Epicentre) | 1 µL |

The procedure is: denaturing at 95°C for 5 min, and incubating at 55°C for 2h.
2. Extending and Ligase Ligating Reaction: adding the following reagents in the above hybridized product (see Table 2), performing the extending and ligation reaction, and the reaction procedure being incubating at 55°C for 1h.

**Table 2 Reagent Table Added During Extending and Ligase Ligating Reaction in Embodiment**

| Reagent | Volume |
|---|---|
| Purified Water | 1.4µL |
| 1 mM dNTPs (NEB) | 1µL |
| 5 U/µL Ampligase DNA ligase (Epicentre) | 1µL |
| 10 × Ampligase Buffer (Epicentre) | 0.5µL |
| 50 mM NAD⁺(NEB) | 0.1µL |
| Phusion® High-Fidelity DNA Polymerase(NEB) | 1µL |

3. Exonuclease Digesting Linear Probe and Nucleic Acid: digesting the remaining probe and the template DNA using an exonuclease. The following reagents are added to the above product (see Table 3), and incubating is performed at 37°C for 40min and incubating is performed at 95°C for 5min.

**Table 3 Reagent Table Added during Reaction of Exonuclease Digesting Linear Probe and Nucleic Acid in Embodiment 1**

| Reagent | Volume |
|---|---|
| Exonuclease I(NEB) | 0.5µL |
| Exonuclease III(NEB) | 0.5µL |
| Lambda Exonuclease(NEB) | 0.5µL |

4. PCR Amplifying: adding the following reagents to the above product to perform PCR amplification (see Table 4).

**Table 4 Reagent Table Added During Reaction of PCR Amplification in Embodiment 1**

| Reagent | Volume |
|---|---|
| 2 × iProof HF Master Mix(Bio-Rad) | 50µL |
| Purified Water | 29.5µL |
| 20 µM Tag1 Primer | 2µL |
| 20 µM Tag2 Primer | 2µL |

The Tag1 Primer Sequence is as shown by SEQ ID NO:9: 
5'CAAGCAGAAGACGGCATACGAGATCTAGTACGGTCTCGTGGGCTCGGAGATGTGT
ATAAGAGACA-s-G3'
The Tag2 Primer Sequence is as shown by SEQ ID NO:10:
   5'AATGATACGGCGACCACCGAGATCTACACATAGAGGCACACTCTTTCCCTACACG ACGCTCTTCCGATC-s-T3', the underlined portion is an index sequence and is changeable.

Pre-denaturing is performed at 98°C for 30s; denaturing is performed at 98°C for 10s, annealing is performed at 55°C for 30s, the extending is performed at 72°C for 30s, and the above procedure is repeated for 30 cycles; the extending is performed at 72°C for 2min, and thermal insulation is performed at 4°C.
5. Library Purifying: purifying the library using Agencourt AMPure XP magnetic beads after the amplification is completed.
6. Sequencing: quality control is performed on the library and an Illumina sequencer, and the sequencing is performed by an Illumina sequencer. Please refer to Table 5 for sequencing results.

**Table 5 Sequencing Result Table of Embodiment 1**

| Experiment Group | Number of Sequences | Comparison Rate | Cover Degree |
|---|---|---|---|
| 1 | 852,746 | 92.04% | 100% |

It can be seen from Table 5 that the cover degree is 100%, which means that the target regions are all captured and enriched, and the comparison rate is greater than 90%, which means that the present invention has higher specificity.

### Embodiment 2: targeting to capture human BRCA, EGFR genes using an optimized procedure.

In the embodiment, five pairs of probes of the target human *BRCA, EGFR* genes, the probes are mixed in equal moles, and the optimized procedure is adding an enzyme reagent and a PCR reagent to a reaction tube simultaneously. The probe structure is as shown in FIG. 1.
1. Hybridizing Probe With Nucleic Acid Sample:
A probe sequence is provided as follows:
P3-a (extension probe) as shown by SEQ ID NO:11: 
5'A-s-C-s-A-s-C-s-T-s-C-s-T-s-T-s-T-s-C-s-C-s-C-s-T-s-A-s-C-s-A-s-C-s-GACGCTCTTCCG
ATCTNNNATTTTTTTAAAGCTAAAGTATTGACTCTTTAAATGTTAAACAAAAATACT3'
P3-b (connection probe) as shown by SEQ ID NO:12: 
5'P-GATACCACAGATAAGCTACTTTTTGAAAAACTGACACTAAATATAACTCT
NNNCTGTCTCTTATACAC-s-A-s-T-s-C-s-T-s-C-s-C-s-G-s-A-s-G-s-C-s-C-s-C-s-A-s-C-s-G-s-A
-s-G-s-A-s-C3'
P4-a (extension probe) as shown by SEQ ID NO:13: 
5'C-s-C-s-C-s-T-s-A-s-C-s-ACGACGCTCTTCCGATCTNNNNTGAGGGCTGAGGTGACC
CTTG3'
P4-b (connection probe) as shown by SEQ ID NO:14: 
5'P-AGATCAAAGTGCTGGGCTCCGGNNNNCTGTCTCTTATACACATCTCCGAGCCCA
CNH2C63'
P5-a (extension probe) as shown by SEQ ID NO:15: 
5'A-s-T-s-C-s-C-s-C-s-C-s-T-s-A-s-C-s-ACGACGCTCTTCCGATCTNNNNGACCATGAGA
GGCCCTGCGG3'
P5-b (connection probe) as shown by SEQ ID NO:16: 
5'P-TCAAGAGAGCTTGGTTGGGAGCTNNNNCTGTCTCTTATACACATCTCCGAGCCC
ACP3'
P6-a (extension probe) as shown by SEQ ID NO:17:
   5'SpC3CCCTACACGACGCTCTTCCGATCTNNNNTCACTGGGCAGCATGTGGCA3'
P6-b (connection probe) as shown by SEQ ID NO:18:

5'P-AGAGAAGCAACATCTCCGAAAGCCANNNNCTGTCTCTTATACACATCTCCGAG
CCCACSpC3 3'

s denotes phosphorothioate modification, P denotes phosphorylation modification, SpC3 denotes a type of spacer arm, NH2C6 denotes amino-modification, N denotes any base of A, T, C or G, an underlined portion is an Illumina sequencing tag sequence 1, NNN in P3-a denotes a UID sequence 1 region, and NNN in P3-b denotes a UID sequence 2 region. NNNN in P4-a denotes a UID sequence 1 region, and NNNN in P4-b denotes a UID sequence 2 region. NNNN in P5-a denotes a UID sequence 1 region, and NNNN in P5-b denotes a UID sequence 2 region. NNNN in P6-a denotes a UID sequence 1 region, and NNNN in P6-b denotes a UID sequence 2 region. A portion underlined with a dotted line is an extension arm, a portion underlined with a double line is a ligation arm, and a portion underlined with a wavy line is an Illumina sequencing tag sequence 2. 17 phosphorothioate modifications in P3-a are a protection structure 1 region, and 19 phosphorothioate modifications in P3-b are a protection structure 2 region. 6 phosphorothioate modifications in P4-a are a protection structure 1 region, and NH2C6 in P4-b is a protection structure 2 region. 9 phosphorothioate modifications in P5-a are a protection structure 1 region, and phosphorylation modification in P5-b is a protection structure 2 region. SpC3 in P6-a is a protection structure 1 region, and SpC3 in P6-b is a protection structure 2 region.
Hybridization reaction system is as shown in Table 6.

**Table 6 Hybridization Reaction System of Embodiment 2**

| Reagent | Volume |
|---|---|
| Purified Water | 6µL |
| 50 ng/µL H1650 cell line DNA | 2µL |
| 3 × 10⁻³ µM probe mixture (i.e., a mixture of SEQ ID NO:11-18) | 1µL |
| 10 × Ampligase Buffer (Epicentre) | 1µL |

The procedure is: denaturing at 95°C for 5 min, and incubating at 55°C for 2h.
2. Extending and Ligase Ligating Reaction: adding the following reagents in the above hybridized product, performing the extending and ligation reaction, and the reaction procedure being incubating at 55°C for 1h.

**Table 7 Reagent Table Added during Reaction of Exonuclease Digesting Linear Probe and Nucleic Acid in Embodiment 2**

| Reagent | Volume |
|---|---|
| Purified Water | 1.4µL |
| 1 mM dNTPs (NEB) | 1µL |
| 5 U/µL Ampligase DNA ligase (Epicentre) | 1µL |
| 10 × Ampligase Buffer (Epicentre) | 0.5 µL |
| 50 mM NAD⁺(NEB) | 0.1µL |
| Phusion® High-Fidelity DNA Polymerase(NEB) | 1µL |

3. Exonuclease Digesting Linear Probe and Nucleic Acid and PCR Amplifying: adding the following reagents to the above product to digest the remaining probe and the template DNA, and performing the PCR amplification:

**Table 8 Reagent Table Added during Reaction of Exonuclease Digesting Linear Probe and Nucleic Acid and PCR Amplification in Embodiment 2**

| Reagent | Volume |
|---|---|
| Exonuclease I(NEB) | 0.5µL |
| Exonuclease III(NEB) | 0.5µL |
| Lambda Exonuclease(NEB) | 0.5µL |
| 2 × iProof HF Master Mix(Bio-Rad) | 50µL |
| Purified Water | 29.5µL |
| 20 µM Tag3 Primer | 2µL |
| 20 µM Tag4 Primer | 2µL |

The reaction procedure is incubating at 37°C for 40min, incubating at 95°C for 5min, and pre-denaturing at 98°C for 30s. Denaturing is performed at 98°C for 10s, annealing is performed at 60°C for 30s, the extending is performed at 72°C for 30s, and the above procedure is repeated for 27 cycles. The extending is performed at 72°C for 2min, and thermal insulation is performed at 4°C.
The Tag3 Primer Sequence is as shown by SEQ ID NO:19: 
5'C-s-AAGCAGAAGACGGCATACGAGATTTCTGCCTGTCTCGTGGGCTCGGAGATGT
GTATAAGAGACA-s-G3'
The Tag4 Primer Sequence is as shown by SEQ ID NO:20:
   5'A-s-ATGATACGGCGACCACCGAGATCTACACTATAGCCTACACTCTTTCCCTACAC GACGCTCTTCCGATC-s-T3', and the underlined portion is an index sequence and is changeable.

Pre-denaturing is performed at 98°C for 30s; denaturing is performed at 98°C for 10s, annealing is performed at 60°C for 30s, the extending is performed at 72°C for 30s, and the above procedure is repeated for 30 cycles; the extending is performed at 72°C for 2min, and thermal insulation is performed at 4°C.
4. Purifying the library using Agencourt AMPure XP magnetic beads after the amplification is completed.
5. Quality control is performed on the library and the sequencing is performed by an Illumina sequencer. Please refer to Table 9 for sequencing results.

**Table 9 Experimental Result Table of Embodiment 2**

| Experiment Group | Number of Sequences | Comparison Rate | Cover Degree |
|---|---|---|---|
| 1 | 846,728 | 91.16% | 100% |

It can be seen from the results of Table 9 that the cover degree is 100%, which means that the target regions are all captured and enriched, and the comparison rate is greater than 90%, which means that the present invention has higher specificity.

### Embodiment 3: targeting to capture human EML4 exon 6 ins 33; ALK exon 20 fusion gene

In the embodiment, a pair of probes of the target human EML4 exon 6 ins 33; ALK exon 20 fusion gene is used, and the probe structure is as shown in FIG. 1.
1. Hybridizing Probe With Nucleic Acid Sample: A probe sequence is provided as follows:
P7-a (extension probe) as shown by SEQ ID NO:21: 
5'G-s-A-s-T-s-C-s-A-s-G-s-T-s-ACGACGCTCTTCCGATCTNNNNNGAAGAGCAGTGAAT
GGGGAATAA 3'
P7-b (connection probe) as shown by SEQ ID NO:22:

5'P-TTATTCCCCATTCACTGCTCTTCNNNNNCTGTCTCTTATACACATCTCCGA-s-T-s-
G-s-G-s-A-s-T-s-T-s-G3'

Where s denotes phosphorothioate modification; an underlined portion is an Illumina sequencing tag sequence 1; NNNNN in P7-a denotes a UID sequence 1 region, a portion underlined with a dotted line is an extension arm; NNNNN in P7-b denotes a UID sequence 2 region; a portion underlined with a double line is a ligation arm; a portion underlined with a wavy line is an Illumina sequencing tag sequence 2; and 7 phosphorothioate modifications in P7-a are a protection structure 1 region, and 7 phosphorothioate modifications in P1-b are a protection structure 2 region.
Hybridization reaction system is as shown in Table 10:

**Table 10 Hybridization Reaction System of Embodiment 3**

| Reagent | Volume |
|---|---|
| Purified Water | 6µL |
| 50 ng/µL H2228 cell line DNA | 2µL |
| 3 × 10⁻³µM probe (i.e., a mixture of SEQ ID NO:21-22) | 1µL |
| 10 × Ampligase Buffer (Epicentre) | 1µL |

The procedure is denaturing at 95°C for 5min, and incubating at 60°C for 2h.
2. Extending and Ligase Ligating Reaction: adding the following reagents in the above hybridized product, performing the extending and ligation reaction, and the reaction procedure being incubating at 60°C for 1h.

**Table 11 Reagent Table Added During Extending and Ligase Ligating Reaction in Embodiment 3**

| Reagent | Volume |
|---|---|
| Purified Water | 1.4µL |
| 1 mM dNTPs (NEB) | 1µL |
| 5 U/µL Ampligase DNA ligase (Epicentre) | 1µL |
| 10 × Ampligase Buffer (Epicentre) | 0.5µL |
| 50 mM NAD⁺(NEB) | 0.1µL |
| Phusion® High-Fidelity DNA Polymerase(NEB) | 1µL |

3. Exonuclease Digesting Linear Probe and Nucleic Acid: digesting the remaining probe and the template DNA using exonuclease. The following reagents are added to the above product:

**Table 12 Reagent Table Added during Reaction of Exonuclease Digesting Linear Probe and Nucleic Acid in Embodiment 3**

| Reagent | Volume |
|---|---|
| Exonuclease I(NEB) | 0.5µL |
| Exonuclease III(NEB) | 0.5µL |
| Lambda Exonuclease(NEB) | 0.5µL |

The incubating is performed at 37°C for 40min, and the incubating is performed at 95°C for 5min.
4. PCR Amplifying: adding the following reagents to the above product to perform PCR amplification.

**Table 13 Reagent Table Added During PCR Amplification Reaction in Embodiment 3**

| Reagent | Volume |
|---|---|
| 2× iProof HF Master Mix(Bio-Rad) | 50µL |
| Purified Water | 29.5µL |
| 20 µM Tag5 Primer | 2µL |
| 20 µM Tag6 Primer | 2µL |

The Tag5 Primer Sequence is as shown by SEQ ID NO:9: 
5'CAAGCAGAAGACGGCATACGAGATGCTCAGGAGTCTCGTGGGCTCGGAGATGTGT
ATAAGAGACA-s-G3'
The Tag6 Primer Sequence is as shown by SEQ ID NO:10:
   5'AATGATACGGCGACCACCGAGATCTACACGGCTCTGAACACTCTTTCCCTACACG ACGCTCTTCCGATC-s-T3', the underlined portion is an index sequence and is changeable.

Pre-denaturing is performed at 98°C for 30s; denaturing is performed at 98°C for 10s, annealing is performed at 60°C for 30s, the extending is performed at 72°C for 30s, and the above procedure is repeated for 30 cycles; the extending is performed at 72°C for 2min, and thermal insulation is performed at 4°C.
5. Library Purifying: purifying the library using Agencourt AMPure XP magnetic beads after the amplification is completed.
6. Quality control is performed on the library and sequencing is performed by an Illumina sequencer. Experimental results are shown in Table 13.

**Table 13 Experimental Result Table of Embodiment 3**

| Experiment Group | Number of Sequences | Comparison Rate | Cover Degree |
|---|---|---|---|
| Group 1 | 617,342 | 97.36% | 100% |

It can be seen from Table 13 that the cover degree is 100%, which means that the target regions are all captured and enriched, and the comparison rate is greater than 97%, which means that the present invention has higher specificity.

### Embodiment 4: targeting to capture human EML4 exon 13; ALK exon 20 fusion gene

In the embodiment, the template is RNA, a pair of probes of the target human EML4 exon 13; ALK exon 20 fusion gene type is used, and the probe structure is as shown in FIG. 1.
1. Hybridizing Probe With Nucleic Acid Sample: A probe sequence is provided as follows:
P8-a (extension probe) as shown by SEQ ID NO:23:
   5'_G-s-T-s-A-s-ACGACGCTCTTCCGATCTNNNNNGCTACTGTAGAGCCCACACCTG 3'
P8-b (connection probe) as shown by SEQ ID NO:24:

5'P-ATGCAGATGGAGCTGCAGANNNNNCTGTCTCTTATACACATCTCCGA-s-C-s-G-s-
T3'

Where s denotes phosphorothioate modification; an underlined portion is an Illumina sequencing tag sequence 1; NNNNN in P8-a denotes a UID sequence 1 region, a portion underlined with a dotted line is an extension arm; NNNNN in P8-b denotes a UID sequence 2 region; a portion underlined with a double line is a ligation arm; a portion underlined with a wavy line is an Illumina sequencing tag sequence 2; and 3 phosphorothioate modifications in P8-a are a protection structure 1 region, and 3 phosphorothioate modifications in P8-b are a protection structure 2 region.
Reverse transcription primer P9 is: GACCGACTACAACCCCAACT SEQ ID NO:25.
Reverse transcription reaction system is as shown in Table 14:

**Table 14 Reverse Transcription Reaction System of Embodiment 4**

| | |
|---|---|
| 50 ng H3122 RNA | 1 |
| 5×M-MLV Buffer | 2 |
| Reverse Transcription Primer P9 (10 uM) | 1 |
| dNTP Mixture(1 0 mM) | 0.5 |
| RTase M-MLV(RNase H-)(200 U/µl) | 0.5 |
| RNase Free H₂O | 5 |

The procedure is incubating at 42°C for 1h.
The reverse transcription product was purified with 1.8X magnetic beads and eluted with 10 µl of purified water.
Hybridization reaction system is as shown in Table 15:

**Table 15 Hybridization Reaction System of Embodiment 4**

| Reagent | Volume |
|---|---|
| Reverse Transcription Purified Product | 8µL |
| 3 × 10⁻³µM probe (i.e., a mixture of SEQ ID NO:23-24) | 1µL |
| 10 × Ampligase Buffer (Epicentre) | 1µL |

The procedure is denaturing at 95°C for 5min, and incubating at 60°C for 2h.
2. Extending and Ligase Ligating Reaction: adding the following reagents in the above hybridized product, performing the extending and ligation reaction, and the reaction procedure being incubating at 60°C for 1h.

**Table 16 Reagent Table Added During Extending and Ligase Ligating Reaction in Embodiment 4**

| Reagent | Volume |
|---|---|
| Purified Water | 1.4µL |
| 1 mM dNTPs (NEB) | 1µL |
| 5 U/µL Ampligase DNA ligase (Epicentre) | 1µL |
| 10 × Ampligase Buffer (Epicentre) | 0.5µL |
| 50 mM NAD⁺(NEB) | 0.1µL |
| Phusion® High-Fidelity DNA Polymerase(NEB) | 1µL |

3. Exonuclease Digesting Linear Probe and Nucleic Acid: digesting the remaining probe and the template DNA using exonuclease. The following reagents are added to the above product:

**Table 17 Reagent Table Added during Reaction of Exonuclease Digesting Linear Probe and Nucleic Acid in Embodiment 4**

| Reagent | Volume |
|---|---|
| Exonuclease I(NEB) | 0.5µL |
| Exonuclease III(NEB) | 0.5µL |
| Lambda Exonuclease(NEB) | 0.5µL |

The incubating is performed at 37°C for 40min, and the incubating is performed at 95°C for 5min.
4. PCR Amplifying: adding the following reagents to the above product to perform PCR amplification.

**Table 18 Reagent Table Added During PCR Amplification Reaction in Embodiment 4**

| Reagent | Volume |
|---|---|
| 2× iProof HF Master Mix(Bio-Rad) | 50µL |
| Purified Water | 29.5µL |
| 20 µM Tag5 Primer | 2µL |
| 20 µM Tag6 Primer | 2µL |

The Tag5 Primer Sequence is as shown by SEQ ID NO:9: 
5'CAAGCAGAAGACGGCATACGAGATGCTCAGGAGTCTCGTGGGCTCGGAGATGTG
TATAAGAGACA-s-G 3'
The Tag6 Primer Sequence is as shown by SEQ ID NO:10:
   5'AATGATACGGCGACCACCGAGATCTACACGGCTCTGAACACTCTTTCCCTACACG ACGCTCTTCCGATC-s-T3', the underlined portion is an index sequence and is changeable.

Pre-denaturing is performed at 98°C for 30s; denaturing is performed at 98°C for 10s, annealing is performed at 60°C for 30s, the extending is performed at 72°C for 30s, and the above procedure is repeated for 32 cycles; the extending is performed at 72°C for 2min, and thermal insulation is performed at 4°C.
5. Library Purifying: purifying the library using Agencourt AMPure XP magnetic beads after the amplification is completed.
6. Quality control is performed on the library and sequencing is performed by an Illumina sequencer. Experimental results are shown in Table 19.

**Table 19 Experimental Result Table of Embodiment 4**

| Experiment Group | Number of Sequences | Comparison Rate | Cover Degree |
|---|---|---|---|
| Group 1 | 652,783 | 96.54% | 100% |

It can be seen from Table 19 that the cover degree is 100%, which means that the target regions are all captured and enriched, and the comparison rate is greater than 96%, which means that the present invention has higher specificity.

Although the embodiments of the present invention have been shown and described above, it should be understood that the above embodiments are only exemplary and cannot be understood as limitations to the present invention. Those ordinarily skilled in the art should understand that changes, modifications, replacements and transformations may be made on the above embodiments within the scope of the present invention without departing from the principles and purposes of the present invention.

## Claims

1. A probe for detecting gene mutations and known and unknown gene fusion types, which is **characterized in** comprising an extension probe and a connection probe, wherein the extension probe consists of following structures:
an extension arm: a sequence complementarily bonded with a nucleic acid template and having a length of 16-50 nt;
a sequencing tag sequence 1 region: used for a Tag1 sequence sequenced on a sequencing platform, and being a sequence or its complementary sequence on transposon Adapters or linkers in any library building kit of the sequencing platform;
a protection structure 1 region: being a 3 to 17 phosphorothioate modifications sequence or a spacer arm, wherein the 3 to 17 phosphorothioate modifications sequence refers to phosphorothioate modifying 3 to 17 bases at a 5' end of the sequencing tag sequence 1 region or phosphorothioate modifying 3 to 17 bases of any section, used for protecting the probe and preventing exonuclease digestion, and preferably being a 3 to 7 phosphorothioate modifications sequence or a spacer arm; and
a UID sequence 1 region further provided between the extension arm of the extension probe and the sequencing tag sequence 1 region, preferably, the UID sequence 1 region consisting of 3 to 12 random bases for distinguishing tag sequences of an original template; and
the connection probe consists of following structures:
a ligation arm: a sequence complementarily bonded with a nucleic acid template and having a length of 16-50 nt, wherein phosphorylation modification is performed on a 5' end of the ligation arm, or phosphorylation modification is voluntarily performed on the 5' end through phosphorylation reaction when the probe is synthesized;
a sequencing tag sequence 2 region: used for a Tag2 sequence sequenced on a sequencing platform, and being a sequence or its complementary sequence on transposon Adapters or linkers in any library building kit of the sequencing platform;
a protection structure 2 region: being a 3 to 19 phosphorothioate modifications sequence or a spacer arm, used for protecting the probe and preventing exonuclease digestion, wherein the 3 to 19 phosphorothioate modifications sequence refers to phosphorothioate modifying 3 to 19 bases at a 3' end of the sequencing tag sequence 2 region or phosphorothioate modifying 3 to 19 bases of any section, and preferably being a 3 to 7 phosphorothioate modifications sequence or a spacer arm.

2. A probe for detecting gene mutations and known and unknown gene fusion types, which is **characterized in** comprising an extension probe and a connection probe, wherein the extension probe consists of following structures:
an extension arm: a sequence complementarily bonded with a nucleic acid template and having a length of 16-50 nt;
a sequencing tag sequence 1 region: used for a Tag1 sequence sequenced on a sequencing platform, and being a sequence or its complementary sequence on transposon Adapters or linkers in any library building kit of the sequencing platform;
a protection structure 1 region: performing 3 to 17 phosphorothioate modifications on a 5' end of the sequencing tag sequence 1 region or introducing a spacer arm so as to protect the 5' end and prevent exonuclease digestion; and
the connection probe consists of following structures:
a ligation arm: a sequence complementarily bonded with a nucleic acid template and having a length of 16-50 nt, wherein phosphorylation modification is performed on a 5' end of the ligation arm, or phosphorylation modification is voluntarily performed on the 5' end through phosphorylation reaction when the probe is synthesized;
a sequencing tag sequence 2 region: used for a Tag2 sequence sequenced on a sequencing platform, and being a sequence or its complementary sequence on transposon Adapters or linkers in any library building kit of the sequencing platform;
a UID sequence 2 region further provided between the ligation arm of the connection probe and the sequencing tag sequence 2 region, preferably, the UID sequence 2 region consisting of 3 to 12 random bases for distinguishing tag sequences of an original template; and
a protection structure 2 region: performing 3 to 19 phosphorothioate modifications on a 3' end of the sequencing tag sequence 2 region, or introducing a spacer arm or a phosphorylation, amino modification so as to protect the 3' end and prevent exonuclease digestion and high-fidelity DNA polymerase digestion.

3. A probe for detecting gene mutations and known and unknown gene fusion types, which is **characterized in** comprising an extension probe and a connection probe, wherein the extension probe consists of following structures:
an extension arm: a sequence complementarily bonded with a nucleic acid template and having a length of 16-50 nt;
a sequencing tag sequence 1 region: used for a Tag1 sequence sequenced on a sequencing platform, and being a sequence or its complementary sequence on transposon Adapters or linkers in any library building kit of the sequencing platform;
a UID sequence 1 region further provided between the extension arm of the extension probe and the sequencing tag sequence 1 region, preferably, the UID sequence 1 region consisting of 3 to 12 random bases for distinguishing tag sequences of an original template; and
a protection structure 1 region: performing 3 to 17 phosphorothioate modifications on a 5' end of the sequencing tag sequence 1 region or introducing a spacer arm so as to protect the 5' end and prevent exonuclease digestion; and
the connection probe consists of following structures:
a ligation arm: a sequence complementarily bonded with a nucleic acid template and having a length of 16-50 nt, wherein phosphorylation modification is performed on a 5' end of the ligation arm, or phosphorylation modification is voluntarily performed on the 5' end through phosphorylation reaction when the probe is synthesized;
a sequencing tag sequence 2 region: used for a Tag2 sequence sequenced on a sequencing platform, and being a sequence or its complementary sequence on transposon Adapters or linkers in any library building kit of the sequencing platform;
a UID sequence 2 region further provided between the ligation arm of the connection probe and the sequencing tag sequence 2 region, preferably, the UID sequence 2 region consisting of 3 to 12 random bases for distinguishing tag sequences of an original template; and
a protection structure 2 region: performing 3 to 19 phosphorothioate modifications on a 3' end of the sequencing tag sequence 2 region, or introducing a spacer arm or a phosphorylation, amino modification so as to protect the 3' end and prevent exonuclease digestion and high-fidelity DNA polymerase digestion.

4. The probe of any one of claims 1 to 3, **characterized in that** the sequencing tag sequence 1 is a sequence from which 0 to 18 bases are reduced at the 5' end of SEQ ID NO:1.

5. The probe of any one of claims 1 to 3, **characterized in that** the sequencing tag sequence 2 is a sequence from which 0 to 16 bases are reduced at the 3' end of a reverse complement sequence of SEQ ID NO:2, wherein the SEQ ID NO:2 is a section of a sequence of Nextera Transposase Adapters Read 2 in Illumina Nextera Library Prep Kits.

6. An application of the probe of any one of claims 1 to 5 to high-throughput sequencing a target-captured target region for detecting known and unknown gene fusion types.

7. A method applicable to high-throughput sequencing a target-captured target region used for detecting gene mutations and known and unknown gene fusion types, which is **characterized in** using the probe of any one of claims 1 to 5; and
preferably, steps being:
hybridizing a probe with a nucleic acid sample;
extending and ligase ligating reaction: after both the extension probe and the connection probe are anchored to a target region of the nucleic acid sample, extending from the 3' end of the extension probe to the 5' end of the connection probe using a polymerizing function of a DNA polymerase, and a ligase ligating to obtain a ligation product;
or simultaneously performing the hybridizing of the probe with the nucleic acid sample, the extending and the ligation reaction;
exonuclease digesting the linear probe and nucleic acid: digesting the probe and the nucleic acid sample by using a single-stranded exonuclease and a double-stranded exonuclease and only leaving the legation product in the previous step, in order to eliminate the effects of the nucleic acid sample and the remaining probe on subsequent PCR amplification;
PCR amplifying, wherein an upstream primer structure sequentially comprises a sequence region for cluster generation, and an index sequence region for sequencing starting from the 5' end; and a full-length sequence region reversely complementary paired with a sequencing Tag sequence 1, wherein preferably, the 5' end, or the 3' end, or the 5' and 3' ends of the upstream primer are phosphorothioate modified; and wherein a downstream primer structure sequentially comprises a sequence region for cluster generation, and an index sequence region for sequencing starting from the 5' end; and a sequencing tag sequence 2 full-length sequence region, wherein preferably, the 5' end, or the 3' end, or the 5' and 3' ends of the downstream primer are phosphorothioate modified;
library purifying: purifying a library by means of magnetic beads, gel extraction after electrophoresis or column purification; and
high-throughput sequencing.

8. The method of claim 7, **characterized in that** in the hybridizing the probe with the nucleic acid sample, the nucleic acid sample is DNA, RNA, cDNA obtained by RNA reverse transcription, a mixture of DNA and RNA, or a mixture of DNR and cDNA.

9. The method of claim 7, **characterized in that** the PCR amplified upstream primer is SEQ ID NO:3, or what is shown by a sequence after the 5' end thereof is phosphorothioate modified, the 3' end thereof is phosphorothioate modified, or the 5' and 3' ends thereof are phosphorothioate modified.

10. The method of claim 7, **characterized in that** the PCR amplified downstream primer is SEQ ID NO:4, or what is shown by a sequence after the 5' end thereof is phosphorothioate modified, or the 3' end thereof is phosphorothioate modified, or the 5' and 3' ends thereof are phosphorothioate modified.


SEQUENCE LISTING

<110> Amoy Diagnostics Co.,LTD.

<120> PROBE AND METHOD FOR HIGH-THROUGHPUT SEQUENCING TARGETED CAPTURE
TARGET REGION USED FOR DETECTING GENE MUTATIONS AS WELL AS KNOWN AND UNKNOWN
GENE FUSION TYPES

<130> ADSW-17003-CNI

<160> 25

<170> PatentIn version 3.5

<210> 1
<211> 33
<212> DNA
<213> Artificial synthesis

<400> 1
acactctttc cctacacgac gctcttccga tct                                    33


<210> 2
<211> 34
<212> DNA
<213> Artificial synthesis

<400> 2
ctgtctctta tacacatctc cgagcccacg agac                                   34


<210> 3
<211> 66
<212> DNA
<213> Artificial synthesis

<400> 3
caagcagaag acggcatacg agatttctgc ctgtctcgtg ggctcggaga tgtgtataag      60

agacag                                                                  66


<210> 4
<211> 70
<212> DNA
<213> Artificial synthesis

<400> 4
aatgatacgg cgaccaccga gatctacaca tagaggcaca ctctttccct acacgacgct      60

cttccgatct                                                              70


<210> 5
<211> 31
<212> DNA
<213> Artificial synthesis


<220>
<221> Phosphorothioate modification
<222> (1)..(3)

<400> 5
acgctcttcc gatctaccct tgtctctgtg t                                    31


<210> 6
<211> 46
<212> DNA
<213> Artificial synthesis


<220>
<221> Phosphorylation
<222> (1)..(1)
<223> A at 5′ end is phosphorylated

<220>
<221> misc_feature
<222> (17)..(28)
<223>  n is a, c, g, or t

<220>
<221> Phosphorothioate modification
<222> (44)..(46)

<400> 6
agcccagagt ccttgcnnnn nnnnnnnnct gtctcttata cacatc                   46


<210> 7
<211> 43
<212> DNA
<213> Artificial synthesis


<220>
<221> Phosphorothioate modification
<222> (1)..(3)

<220>
<221> misc_feature
<222> (16)..(27)
<223>  n is a, c, g, or t

<400> 7
acgctcttcc gatctnnnnn nnnnnnncag catgtggcac cat                      43


<210> 8
<211> 34
<212> DNA
<213> Artificial synthesis


<220>
<221> Phosphorylation
<222> (1)..(1)
<223> A at 5′ end is phosphorylated

<220>
<221> Phosphorothioate modification
<222> (31)..(34)

<400> 8
   accttttctc atgtctctgt ctcttataca catc                                    34

<210> 9
<211> 66
<212> DNA
<213> Artificial synthesis

<220>
<221> Phosphorothioate modification
<222> (66)..(66)

<400> 9
caagcagaag acggcatacg agatctagta cggtctcgtg ggctcggaga tgtgtataag       60

agacag                                                                  66

<210> 10
<211> 70
<212> DNA
<213> Artificial synthesis

<220>
<221> Phosphorothioate modification
<222> (70)..(70)

<400> 10
aatgatacgg cgaccaccga gatctacaca tagaggcaca ctctttccct acacgacgct       60

cttccgatct                                                              70

<210> 11
<211> 86
<212> DNA
<213> Artificial synthesis

<220>
<221> Phosphorothioate modification
<222> (1)..(17)

<220>
<221> misc_feature
<222> (34)..(36)
<223>  n is a, c, g, or t

<400> 11
acactctttc cctacacgac gctcttccga tctnnnattt ttttaaagct aaagtattga       60

ctctttaaat gttaaacaaa aatact                                            86

<210> 12
<211> 87
<212> DNA
<213> Artificial synthesis

<220>
<221> Phosphorylation
<222> (1)..(1)
<223> G at 5′ end is phosphorylated

<220>
<221> misc_feature
<222> (51)..(53)
<223>  n is a, c, g, or t

<220>
<221> Phosphorothioate modification
<222> (69)..(87)

<400> 12
gataccacag ataagctact ttttgaaaaa ctgacactaa atataactct nnnctgtctc      60

ttatacacat ctccgagccc acgagac                                          87


<210> 13
<211> 49
<212> DNA
<213> Artificial synthesis


<220>
<221> Phosphorothioate modification
<222> (1)..(6)

<220>
<221> misc_feature
<222> (25)..(28)
<223>  n is a, c, g, or t

<400> 13
ccctacacga cgctcttccg atctnnnntg agggctgagg tgacccttg                  49


<210> 14
<211> 55
<212> DNA
<213> Artificial synthesis


<220>
<221> Phosphorylation
<222> (1)..(1)
<223> A at 5′ end is phosphorylated

<220>
<221> misc_feature
<222> (23)..(26)
<223>  n is a, c, g, or t

<220>
<221> Amino-modification
<222> (55)..(55)
<223>  NH2C6 is amino modified

<400> 14
agatcaaagt gctgggctcc ggnnnnctgt ctcttataca catctccgag cccac           55

<210> 15
<211> 51
<212> DNA
<213> Artificial synthesis

<220>
<221> Phosphorothioate modification
<222> (1)..(9)

<220>
<221> misc_feature
<222> (28)..(31)
<223>  n is a, c, g, or t

<400> 15
atcccctaca cgacgctctt ccgatctnnn ngaccatgag aggccctgcg g          51

<210> 16
<211> 56
<212> DNA
<213> Artificial synthesis

<220>
<221> Sulfation
<222> (1)..(1)
<223> T at 5′ end is phosphorylated

<220>
<221> misc_feature
<222> (24)..(27)
<223>  n is a, c, g, or t

<220>
<221> Sulfation
<222> (56)..(56)
<223> T at 3′ end is phosphorylated

<400> 16
tcaagagagc ttggttggga gctnnnnctg tctcttatac acatctccga gcccac          56

<210> 17
<211> 48
<212> DNA
<213> Artificial synthesis

<220>
<221> Spacer arm
<222> (1)..(1)
<223> SpC3 spacer arm

<220>
<221> misc_feature
<222> (25)..(28)
<223>  n is a, c, g, or t

<400> 17
ccctacacga cgctcttccg atctnnnntc actgggcagc atgtggca          48

<210> 18
<211> 58
<212> DNA
<213> Artificial synthesis

<220>
<221> Phosphorylation
<222> (1)..(1)
<223> A at 5′ end is phosphorylated

<220>
<221> misc_feature
<222> (26)..(29)
<223>  n is a, c, g, or t

<220>
<221> Spacer arm
<222> (58)..(58)
<223> SpC3 spacer arm

<400> 18
agagaagcaa catctccgaa agccannnnc tgtctcttat acacatctcc gagcccac          58

<210> 19
<211> 66
<212> DNA
<213> Artificial synthesis

<220>
<221> Phosphorothioate modification
<222> (1)..(1)

<220>
<221> Phosphorothioate modification
<222> (66)..(66)

<400> 19
caagcagaag acggcatacg agatttctgc ctgtctcgtg ggctcggaga tgtgtataag          60

agacag                                                                      66

<210> 20
<211> 70
<212> DNA
<213> Artificial synthesis

<220>
<221> Phosphorothioate modification
<222> (1)..(1)

<220>
<221> Phosphorothioate modification
<222> (70)..(70)

<400> 20
aatgatacgg cgaccaccga gatctacact atagcctaca ctctttccct acacgacgct          60

cttccgatct                                                              70


<210> 21
<211> 53
<212> DNA
<213> Artificial synthesis



<220>
<221> Phosphorothioate modification
<222> (1)..(7)

<220>
<221> misc_feature
<222> (26)..(30)
<223>  n is a, c, g, or t

<400> 21
gatcagtacg acgctcttcc gatctnnnnn gaagagcagt gaatggggaa taa              53


<210> 22
<211> 58
<212> DNA
<213> Artificial synthesis



<220>
<221> Sulfation
<222> (1)..(1)
<223> T at 5′ end is phosphorylated

<220>
<221> misc_feature
<222> (24)..(28)
<223>  n is a, c, g, or t

<220>
<221> Phosphorothioate modification
<222> (52)..(58)

<400> 22
ttattcccca ttcactgctc ttcnnnnnct gtctcttata cacatctccg atggattg        58


<210> 23
<211> 48
<212> DNA
<213> Artificial synthesis



<220>
<221> Phosphorothioate modification
<222> (1)..(3)

<220>
<221> misc_feature
<222> (22)..(26)
<223>  n is a, c, g, or t

<400> 23

gtaacgacgc tcttccgatc tnnnnngcta ctgtagagcc cacacctg 48

<210> 24
<211> 50
<212> DNA
<213> Artificial synthesis

<220>
<221> Phosphorylation
<222> (1)..(1)
<223> A at 5′ end is phosphorylated

<220>
<221> misc_feature
<222> (20)..(24)
<223> n is a, c, g, or t

<220>
<221> Phosphorothioate modification
<222> (48)..(50)

<400> 24
atgcagatgg agctgcagan nnnnctgtct cttatacaca tctccgacgt 50

<210> 25
<211> 20
<212> DNA
<213> Artificial synthesis

<400> 25
gaccgactac aaccccaact